# EUROPEAN PATENT APPLICATION

(11) **EP 4 137 865 A1**
(43) Date of publication of application: **22.02.2023**
(21) Application number: 21191876.8
(22) Date of filing: 18.08.2021
(51) Int. Cl.: G02B 21/32, C12M 1/00, C12M 1/36

(54) **AUTOMATED INCUBATION AND MICROSCOPE SYSTEM**

(71) Applicant: Leica Microsystems CMS GmbH, 35578 Wetzlar (DE)
(72) Inventor: Christ, Stefan, 35641 Schöffengrund (DE)
(74) Representative: Schaumburg und Partner Patentanwälte mbB

(57) **Abstract**

A microscope system (100, 300, 600, 700) comprising an enclosed sample chamber (104) configured to receive a sample carrier (306) in an examining position (106) in which a sample arranged on the sample carrier (306) can be microscopically examined. An enclosed incubation chamber (116) is separated from the sample chamber (104) and configured to receive the sample carrier (306) in at least one storing position (118). The microscope system (100, 300, 600, 700) further comprises a sample carrier transfer unit (126) having an enclosed transfer chamber (128) that is connected to the sample chamber (104) by a first opening (130), and to the incubation chamber (116) by a second opening (132), and a sample carrier (306) handling device (134) arranged within the transfer chamber (128). The sample carrier (306) handling device (134) is configured to move the sample carrier (306) between the storing position (118) and the examination position (106).

## Description

### Technical field

The invention relates to a microscope system.

### Background

In microscopy, when working with living samples, incubation is frequently required in order to grow and culture the samples. Most commonly a cell culture incubator is used, which is a standalone instrument that offers control over temperature, CO₂ concentration and humidity inside large and enclosed volume. However, since the cell culture incubator and the microscope used for observing the sample are physically separated, the sample has to be retrieved from the incubator and transferred through a non-incubated and potentially non-sterile atmosphere in order to observe it. This transfer can have detrimental effects on the sample, particularly a sensitive sample might even be irreversible damaged. Further, automated transfer of sample from the incubator to the microscope and back, for example by means of a robotic arm, requires a lot of technical expertise as well as expensive and complex assemblies having a large foot print. This means, that often times manual handling of the samples is required, resulting in a low through put and short walk-away times.

Therefore, there are various solutions that integrate an incubator and a microscope. Most notably for use with conventional microscopes, i.e. open microscopes, are stage top incubators, i.e. sample carriers that provide an incubated atmosphere themselves, and cage incubators, i.e. tent-like structures that are arranged around the microscope. However, these solutions typically require manual handling of the samples. They are therefore unsuited for experiments that require a large number of samples to be examined in quick succession, i.e. experiments that require a high throughput. It is also possible to use the sample space of an enclosed microscope, for example a cage or box type microscope, as an incubator by providing means for controlling the atmosphere inside the sample chamber of the microscope. However, the space for storing samples inside the sample chamber is typically very limited. This solution is therefore equally unsuited for experiments that require a large number of samples to be examined.

### Summary

It is therefore an object to provide a microscope system that allows to perform experiments requiring an incubation and/or (semi) sterile environment with a high throughput and long walk-away times.

The aforementioned object is achieved by the subject-matter of the independent claim. Advantageous embodiments are defined in the dependent claims and the following description.

The proposed microscope system comprises an enclosed sample chamber configured to receive a sample carrier in an examining position in which a sample arranged on the sample carrier can be microscopically examined. An enclosed incubation chamber is separated from the sample chamber and configured to receive the sample carrier in at least one storing position. The microscope system further comprises a sample carrier transfer unit having an enclosed transfer chamber that is connected to the sample chamber by a first opening, and to the incubation chamber by a second opening, and a sample carrier handling device arranged within the transfer chamber. The sample carrier handling device is configured to move the sample carrier between the storing position and the examination position.

When the sample is not being observed, the sample carrier is stored inside the incubation chamber in an enclosed environment. Thereby, the sample carrier does not take up valuable space inside the sample chamber itself while in its storing position. When the sample is to be observed, the sample carrier handling device moves the sample carrier from its storing position inside the incubation chamber via the transfer chamber to the examination position inside the sample chamber. The sample carrier, and thus the sample, remains in an enclosed environment at all times. The enclosed environment comprising the sample chamber, the transfer chamber and the incubation chamber can easily be made into a sterile or semi sterile environment and can provide an incubation atmosphere if the experiment demands it. Thereby, a continuous incubation of the sample is ensured and samples that are sensitive to contamination can be examined with the microscope.

The process of transferring the sample carrier from its storing position to the examining position and back is easily automated, allowing the experiment to be performed without human intervention. Since the space inside the incubation chamber is not as limited as the space inside the sample chamber, more than one storing position can be provided, allowing a large number of sample carriers to be stored inside the incubation chamber. Thus, the proposed microscope allows to perform experiments requiring an incubation and/or (semi) sterile environment with a high throughput and long walk-away times.

The sample carrier may comprise at least one of a microscope slide, a petri dish or a multiwell plate configured to receive multiple samples. The sample carrier may further comprise an interface portion for engaging with the sample carrier handling device. The interface portion may be of a standardized size, for example have the dimensions of a microscope slide or a multiwell plate, and be configured to receive other elements, for example the aforementioned elements, for holding a sample.

In a preferred embodiment, the sample carrier handling device comprises an arm that is movable within the transfer chamber. The arm has an engaging portion configured to engage and disengage with the sample carrier in order to move the sample carrier. The arm is a flexible mechanism for handling the sample carrier. In particular, the arm can be designed such that a number of different sample carriers of various sizes and shapes can be engaged with. Thereby a number of different formfactors of sample carriers can used with the proposed microscope.

In another preferred embodiment, the engaging portion is configured to engage and disengage with an adapter portion of the sample carrier in order to move the sample carrier. In this embodiment, the sample carrier can be securely attached to the arm. This prevents loss of the sample carrier inside the transfer chamber, which would require the user to retrieve the sample carrier and thereby interrupt the experiment.

In another preferred embodiment, the arm is configured to extend and retract along at least one direction. The arm may for example be a telescopic arm or a scissor gripper. In this embodiment, the arm can extend into the sample chamber and/or the incubation chamber for engaging with the sample carrier. When the arm is not needed for moving the sample carrier, the arm can retract into the transfer chamber. Thereby, valuable space is saved.

In another preferred embodiment, the transfer chamber comprises sterilization means configured to disinfect and/or sterilize the microscope system, in particular the sample carrier handling device and the inside of the transfer chamber. Preferably, the sterilization means are configured to disinfect and/or sterilize by means of UV light. By providing the sterilization means the process of disinfecting and/or sterilizing the microscope system can be automated. In particular, the microscope can be disinfected and/or sterilized at regular intervals during a long term experiments, preventing contamination of the stored samples, and thus further reducing the need for human interaction and increasing the walk-away time.

In another preferred embodiment, the first opening and/or the second opening comprise an automatic door, in particular a sliding door. By providing doors between the sample chamber, the transfer chamber, and/or the incubation chamber, the enclosed space defined by those elements is compartmentalized. The compartmentalization creates an airlock between the sample chamber and the incubation chamber, and thereby prevents cross contamination between the two atmospheres.

In another preferred embodiment the microscope system comprises a first fan assembly configured to blow atmosphere into the sample chamber through at least one first opening of the sample chamber, and at least one second opening of the sample chamber. The at least one first opening and the at least one second opening are arranged on opposite sides of the sample chamber, in particular a top side and a bottom side of the sample chamber. This provides a directed flow of atmosphere inside the sample chamber. This flow can entrain any particles, like dust, dirt, and germs, entering the sample chamber, for example when manually manipulation and/or pipetting the sample, and transport them to an exit opening. This allows for a sterile or semi-sterile work environment inside the sample chamber. Further, in case of an incubated sample chamber, it is possible to recycle incubation atmosphere. Incubation atmosphere drained out of the sample chamber may be recycled and blown back into the sample chamber. A part of the incubation atmosphere may be refreshed or replenished. This saves incubation atmosphere volume and energy after opening and closing of the door of the sample chamber.

In another preferred embodiment, the first fan assembly, the at least one first opening and the at least one second opening of the sample chamber are configured to generate a laminar flow inside the sample chamber. The laminar flow acts like a shield or curtain preventing atmosphere from escaping the sample chamber and preventing external atmosphere from entering the sample chamber.

In another preferred embodiment the microscope system comprises a second fan assembly configured to blow atmosphere into the incubation chamber through at least one first opening of the incubation chamber, and at least one second opening of the incubation chamber. The at least one first opening and the at least one second opening are arranged on opposite sides of the incubation chamber, in particular a top side and a bottom side of the incubation chamber. This provides a directed flow of atmosphere in the sample chamber. This flow can entrain any particles, like dust, dirt, and germs, entering the incubation chamber, for example when the user adds new sample carriers to the incubation chamber, and transport them to an exit opening. It is further possible to recycle the atmosphere inside the incubation chamber. Incubation atmosphere drained out of the incubation chamber may be recycled and blown back into the incubation chamber. A part of the incubation atmosphere may be refreshed or replenished. This saves incubation atmosphere volume and energy after opening and closing of the door of the incubation chamber.

In another preferred embodiment, the first fan assembly, the at least one first opening and the at least one second opening are configured to generate a laminar flow inside the incubation chamber. The laminar flow acts like a shield or curtain preventing atmosphere from escaping the incubation chamber and preventing external atmosphere from entering the incubation chamber.

In another preferred embodiment the microscope system comprises at least one sample condition determination unit. The sample condition determination unit has an image acquisition unit arranged inside the incubation chamber and is configured to capture at least one image of the sample carrier. The microscope system may further comprise a control unit configured to determine the condition of one or more samples placed in the sample carrier based on the image. The sample condition determination unit may in particular be used to determine if a cultivated sample is ready for observation, for example when a monolayer has reached a predetermined percentage of confluence. Thereby, the cultivation and the subsequent observation of the sample can be automated. This further enhances the walk-away time of the microscope.

In another preferred embodiment the microscope system comprises a microscope stage arranged below the sample chamber having a receiving portion configured to receive the sample carrier and that defines the examining position. The receiving portion may further be configured to guide the sample carrier towards examining position. This allows the sample carrier handling unit to position the sample carrier exactly on the examining position, thereby allowing a fast and reliable observation of the sample.

In another preferred embodiment the microscope comprises a stage drive configured to laterally move the microscope stage in a plane which is parallel to the top surface of the microscope stage. Preferably, the microscope is an X-Y-table. This allows for example to select an individual cavity of a multiwell plate or selecting a specific region of interest of a single sample for observation.

In another preferred embodiment, the sample chamber, the incubation chamber and/or the transfer chamber contain an incubation atmosphere, a sterile atmosphere and/or a semi sterile atmosphere. The environment comprising the sample chamber, the transfer chamber and the incubation chamber of the sample chamber can be used to provide an incubation atmosphere for continuous incubation of the sample. This allows for example for the long time observation of cell cultures without the need of transferring the sample through a potentially non-favorable environment such as a laboratory environment. This would not only stress the sample but take additional time as well. Further, the enclosed environment can be used to provide a sterile or at least semi-sterile atmosphere. In this embodiment, even samples that are extremely sensitive to contamination can be examined with the microscope.

In another preferred embodiment the microscope system comprises a box-type microscope housing defining the sample chamber, the housing having a door for providing access to the sample chamber. Box-type microscopes comprise a housing in which all the microscopes components are arranged. The housing typically comprises one or more openings for accessing the inside of the microscope. Due to the enclosed or even sealed nature of the housing, box-type microscopes are especially suited for precisely controlling the environment of the samples, for example by a climate control unit. In this embodiment, the sample chamber is located inside the box-type microscope housing. This means, that the environment of the sample chamber can be precisely controlled.

In another preferred embodiment, the box-type microscope housing defines a component space below the microscope stage, the component space including a plurality of microscope components. The component space may include a power source of the microscope, an optical imaging system, an illumination system, filters, an environmental control unit, or an incubation control unit.

In another preferred embodiment, the microscope housing further defines the incubation chamber and the transfer chamber. The housing has a second door for providing access to the incubation chamber. In this embodiment, the sample chamber, the incubation chamber, and the transfer chamber are combined into a single integrated unit. Such an integrated unit requires little to no setup time, since its components are adapted to each other.

In another preferred embodiment the microscope comprises at least one sample carrier identification unit configured to identify the sample carrier. The sample carrier identification unit may be arranged inside the sample chamber or the incubation chamber. In particular, the sample carrier identification unit may be integrated with the sample condition determination unit. The sample carrier identification unit can be used to uniquely identify the sample carrier, and thus the sample or sample contained therein. Thereby, multiple sample carrier can be automatically examined and/or manipulated one after the other. This further enhances the walk-away time of the microscope.

In another preferred embodiment, the sample carrier identification unit comprises a camera, barcode reader and/or RFID-reader. The camera can be used to uniquely identify the sample carrier based on a tag such as a barcode or a QR code, or based on a handwritten label. The barcode reader can be used to uniquely identify the sample carrier based on a barcode. The RFID-reader can be used to uniquely identify the sample carrier based on an RFID-tag.

### Short Description of the Figures

Hereinafter, specific embodiments are described referring to the drawings, wherein:
- Figure 1: is a schematic view of a microscope system according to an embodiment;
- Figure 2: is a schematic view of a microscope system according to another embodiment;
- Figure 3: is a schematic cut view of a microscope system according to another embodiment comprising a single microscope housing;
- Figure 4: is a schematic cut view of a microscope system according to Figure 3;
- Figure 5: is another schematic cut view of a microscope system according to Figure 3;
- Figure 6: is a schematic top view of the sample carrier handling unit and the microscope stage of a microscope system according to an embodiment;
- Figure 7: is a schematic side view of the sample carrier handling unit and the incubation chamber of a microscope system according to an embodiment; and
- Figure 8: is a flowchart of a method for examining samples with the microscope system detailed above.

### Detailed Description

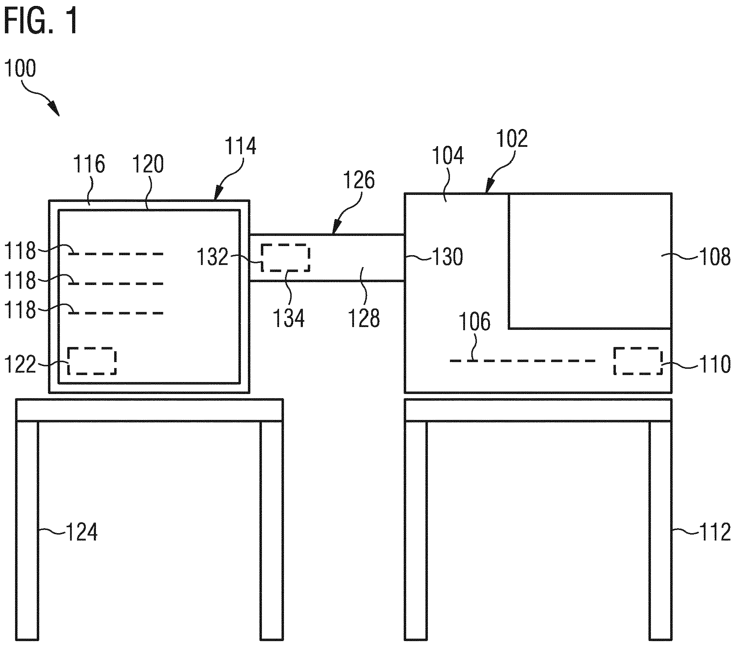

Figure 1 is a schematic view of a microscope system 100 according to an embodiment.

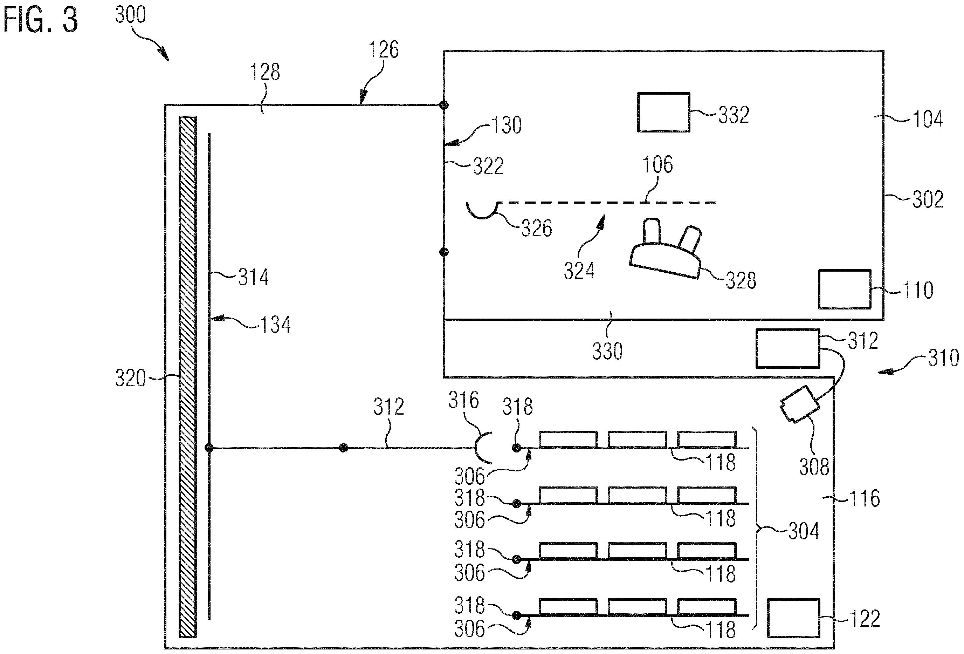

The microscope system 100 comprises a first housing 102 that is exemplary formed as a box-type microscope housing and defines an enclosed sample chamber 104. The sample chamber 104 is configured to receive a sample carrier 306 (c.f. Figure 3) in an examining position 106 for microscopic observation. A door 108 of the first housing 102 provides access to the sample chamber 104, for example for manually loading or retrieving the sample carrier 306 into or from the sample chamber 104, respectively. The sample chamber 104 comprises first climate control unit 110 for controlling the temperature, the CO₂ concentration and the humidity of the incubation atmosphere inside the sample chamber 104. Thereby, an incubation atmosphere is provided for the sample in the examining position 106. The sample chamber 104 and further elements of the first housing 102 will be described in more detail below with reference to Figures 3 to 7. The first housing 102 is exemplary arranged on a first optical table 118 that is not part of the microscope system 100 itself.

A second housing 114 of the microscope system 100 defines an enclosed incubation chamber 116. The incubation chamber 116 is exemplary formed as a cell culture incubator and defines one or more storing positions 118 for storing sample carriers 306. A door 120 of the second housing 114 provides access to the incubation chamber 116, for example for manually loading or retrieving the sample carrier 306 into or from the incubation chamber 116, respectively. The incubation chamber 116 is configured to provide an incubation atmosphere. A second climate control unit 122 of the incubation chamber 116 provides means for controlling the temperature, the CO₂ concentration and the humidity of the incubation atmosphere inside the incubation chamber 116. Thereby, it is possible to culture and grow samples stored inside the incubation chamber 116. The incubation chamber 116 and further elements of the second housing 114 will be described in more detail below with reference to Figures 3 to 7. The second housing 114 is exemplary arranged on a second optical table 124 that is not part of the microscope system 100 itself.

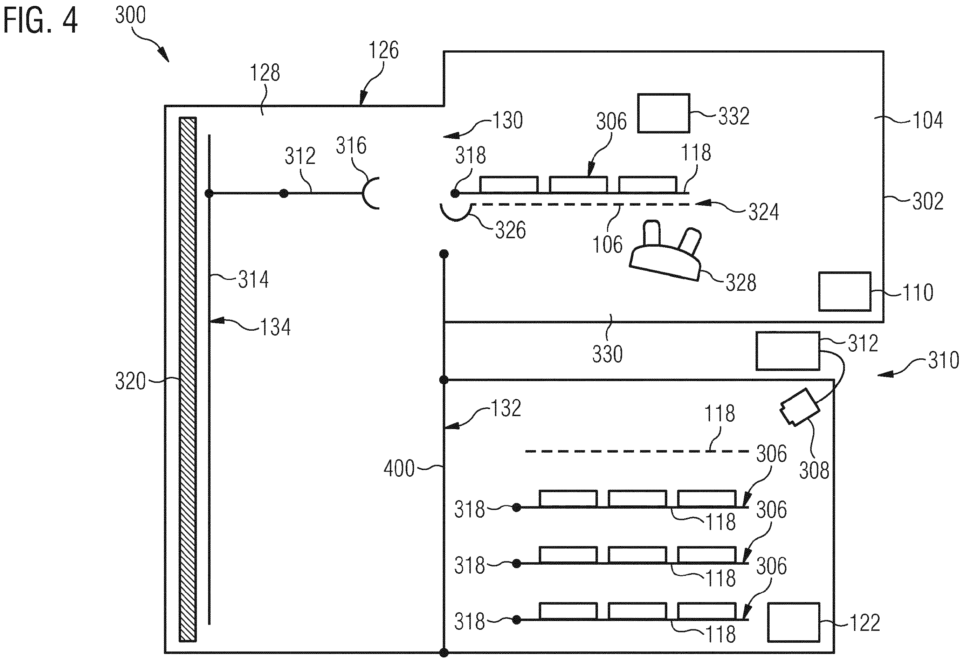

The microscope system 100 further comprises a sample carrier transfer unit 126 for moving the sample carrier 306 between the storing position 118 and the examination position 106. The sample carrier transfer unit 126 comprises a transfer chamber 128 that is arranged between the first and second housings 102, 114. The transfer chamber 128 is connected to the sample chamber 104 by a first opening 130, and to the incubation chamber 116 by a second opening 132. The first and second openings 130, 132 can be closed by means of automatic doors 322, 400 (c.f. Figures 3 and 4). The transfer chamber 128 may comprise a third climate control unit similar to the climate control units 110, 122 of the incubation chamber 116 and the sample chamber 104. The sample carrier transfer unit 126 further comprises a sample carrier handling unit 134 that is configured to engage with the sample carrier in order to move the sample carrier 306. The sample carrier handling unit 134 will be described in more detail below with reference to Figures 3 to 7.

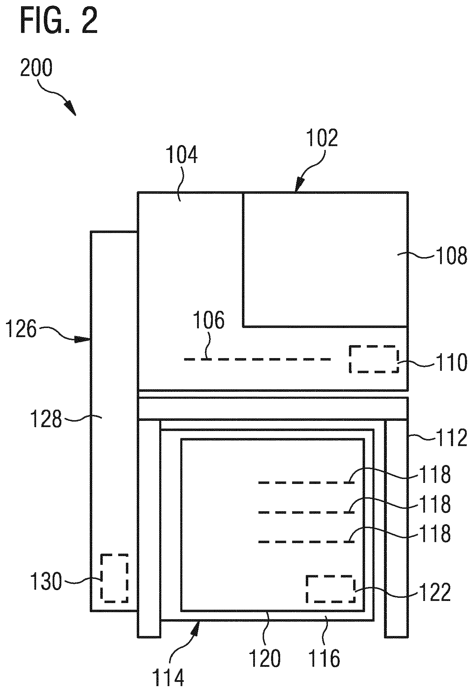

Figure 2 is a schematic view of a microscope system 200 according to another embodiment.

The microscope system 200 according to Figure 2 is distinguished from the microscope system 100 according to Figure 1 in that the second housing 114 comprising the incubation chamber 116 is arranged below the first housing 102 comprising the sample chamber 104. The microscope system 200 according to Figure 2 is further distinguished from the microscope system 100 according to Figure 1 in that the transfer chamber 128 is arranged to the left side of the first and second housing 114 in Figure 2.

The microscope system 100 according to Figure 1 and the microscope system 200 according to Figure 2 exemplary comprise two housings 102, 114 that define the sample chamber 104 and the incubation chamber 116, respectively. The transfer chamber 128 is arranged outside the first and second housings 102, 114. However, it is also possible to integrate the sample chamber 104, the transfer chamber 128 and the incubation chamber 116 into a single housing.

Figure 3 is a schematic cut view of a microscope system 300 according to another embodiment comprising a single microscope housing 302.

The incubation chamber 116 is located in a lower part of the microscope housing 302. Inside the incubation chamber 116 is a shelf-like structure 304 that defines storing positions 118 for four sample carriers 306. A camera 308 is located at the top of the incubation chamber 116. The camera 308 is part of a sample condition determination unit 310. The sample condition determination unit 310 further comprises a control unit 312 that is configured to determine the condition of one or more samples placed the sample carriers 306 based an image captured by the camera 308.

The transfer chamber 128 of the sample carrier transfer unit 126 is located to the left of the incubation chamber 116 in Figure 3. The sample carrier handling device 134 of the sample carrier transfer unit 126 is arranged inside the transfer chamber 128. The sample carrier handling device 134 comprises an arm 312 for engaging with the sample carrier 306 and a guide rail 314 for moving the arm 312 along a vertical direction. The arm 312 can extend into the incubation chamber 116 and the sample chamber 104, respectively. In Figure 3 the arm 312 is shown in a lower position and extended into the incubation chamber 116. An engaging portion 316 is arranged at the end of the arm 312 that is reaching into the incubation chamber 116. The engaging portion 316 can engage with an adapter portion 318 of the sample carriers 306 in order to secure the sample carrier 306 to the arm 312 for movement.

The sample carrier transfer unit 126 further comprises sterilization means 320 for disinfecting and/or sterilizing the inside of the transfer chamber 128. The sterilization means 320 are exemplary formed as an UV lamp. The sterilization means 320 are used to prevent contamination of transfer chamber 128 and the samples. Since UV light for disinfecting and/or sterilizing is used in this embodiment and most sample carriers 306 are transparent to UV light, the sterilization means 320 are preferably used when no sample carrier 306 is inside the transfer chamber 128.

The sample chamber 104 is located in an upper part of the microscope housing 302 to the right of the transfer chamber 128 in Figure 3. A first door 322 arranged inside the first opening 130 between the sample chamber 104 and the transfer chamber 128 is closed in Figure 3. The sample chamber 104 comprises a microscope stage 324 having a receiving portion 326 for receiving one or more of the sample carriers 306. Thereby, the microscope stage 324 defines the examining position 106. The microscope stage 324 is movable along two orthogonal directions, i.e. the microscope stage 324 is a so called X-Y-table. Thus, moving the microscope stage 324 allows for example for selecting an individual cavity or well of the sample carrier 306 or selecting a specific region of interest of a single sample for observation.

The microscope according to Figure 3 is exemplary formed as a transmitted light microscope. Imaging optics 328 for imaging the samples are exemplary arranged below the microscope stage 324, in particular below the examining position 106, in a component space 330 and an illumination system 332 for illuminating the samples is arranged above the microscope stage 324 inside the sample chamber 104. The optical axis of the Imaging optics 328 and the optical axis of the illumination system 332 are aligned. Thereby, illumination light emitted by the illumination system 332 passes through the samples before entering the Imaging optics 328. In an alternative embodiment, the positions of the Imaging optics 328 and the illumination system 332 may be reversed. In another alternative embodiment, both the imaging optics 328 and the illumination system 332 are arranged on the same side of the microscope stage 324. Additional components such as a power source or various filters may be arranged in the component space 330.

Figure 4 is a schematic cut view of a microscope system 300 according to Figure 3.

In Figure 4 one of the sample carrier 306 is arranged atop the microscope stage 324. The arm 312 of the sample carrier 306 handling unit 134 is in an upper position and retracted into the transfer chamber 128 after having moved the sample carrier 306 from the storing position 118 to the sample chamber 104. A second door 400 arranged inside the second opening 132 between the incubation chamber 116 and the transfer chamber 128 is closed in Figure 4.

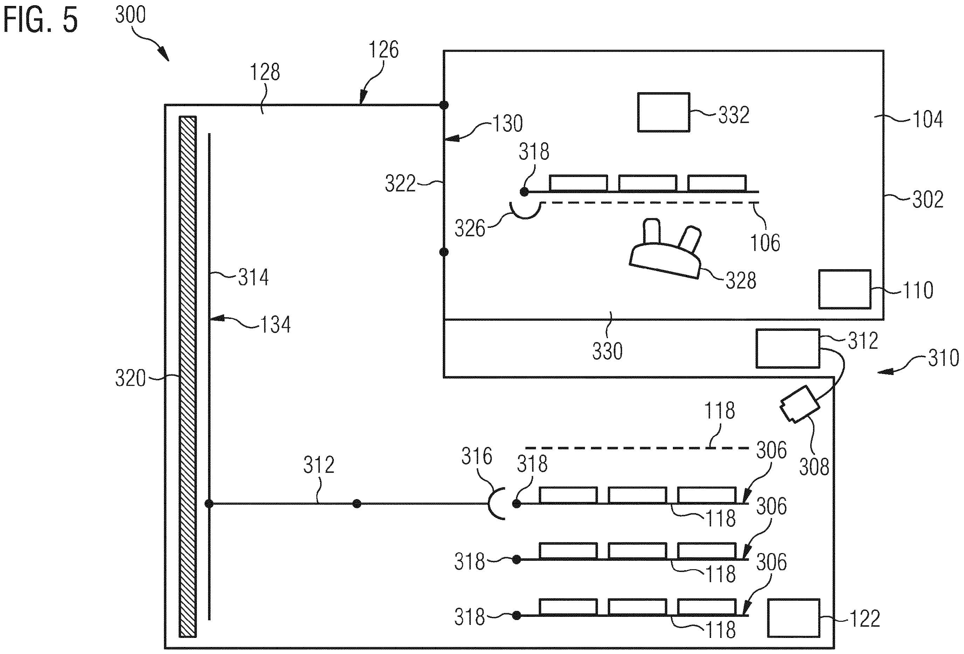

Figure 5 is a schematic cut view of a microscope system 300 according to Figure 3. In Figure 5 the microscope stage 324 has been moved to the right in Figure 5 relative to its position shown in Figure 4. The sample carrier 306 is in its examining position 106 for observing one or more sample arranged therein by means of the imaging optics 328. The first door 322 between the sample chamber 104 and the transfer chamber 128 is closed.

Figure 3 to 5 illustrate how the sample carrier transfer unit 126 moves sample carriers 306 from their storing position 118 inside the incubation chamber 116 to the examining position 106 inside the sample chamber 104. Whether or not a particular sample carrier 306 is to be moved, can be determined by a user input or by automation, in particular based on an input from the sample condition determination unit 310.

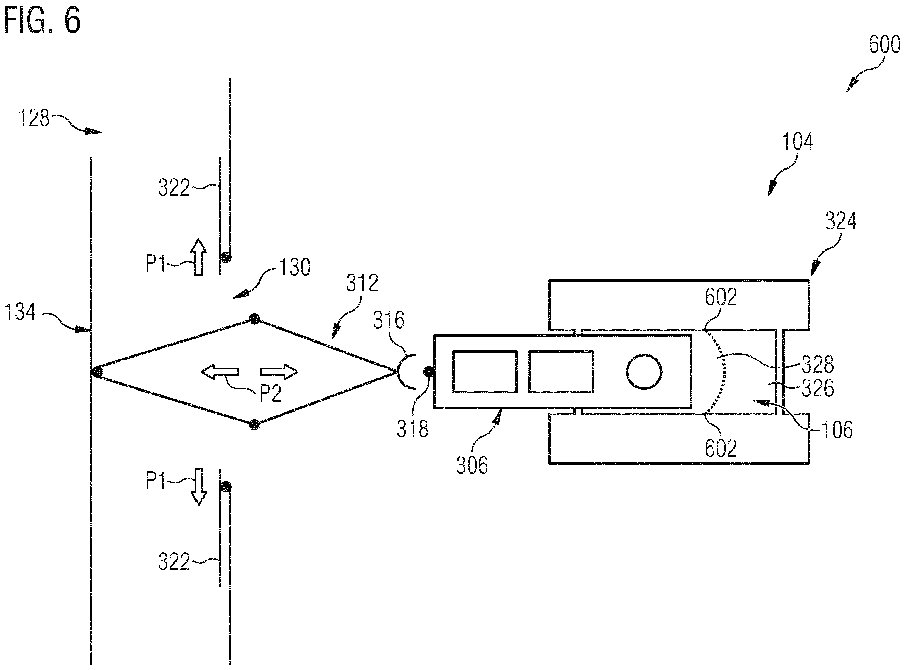

Figure 6 is a schematic top view of the sample carrier 306 handling unit 134 and the microscope stage 324 of a microscope system 600 according to an embodiment.

Figure 6 also shows the first opening 130 between the transfer chamber 128 which is located to the left in Figure 6, the sample chamber 104 which is located to the right in Figure 6, and the first door 322, which is arranged in the first opening 130. The first door 322 is exemplary formed as a sliding door comprising two parts which can be moved in opposite direction in order to open the door. The moved of the two parts of the first door 322 is indicated in Figure 6 by a first set of arrows P1.

As can be seen in Figure 6, the arm 312 of the sample carrier 306 handling unit 134 comprises a scissor-like mechanism that allows the arm 312 to extend and retract. In Figure 6 the arm 312 is exemplary shown to be extended into the sample chamber 104 and to be engaged with the sample carrier 306 arranged within the receiving portion 326 of the microscope stage 324. The extension and retraction of the arm 312 is indicated in Figure 6 by a second set of arrows P2.

The receiving portion 326 of the microscope stage 324 according to the present comprises two guiding portions that limit the movement of the sample carrier 306 perpendicular to the direction of extension of the arm 312. This way, the sample carrier 306 is guide into its examining position 106, when the arm 312 of the sample carrier 306 handling unit 134 is extended. The examining position 106 is located above the imaging optics 328 which are shown in Figure 6 as a dotted circle.

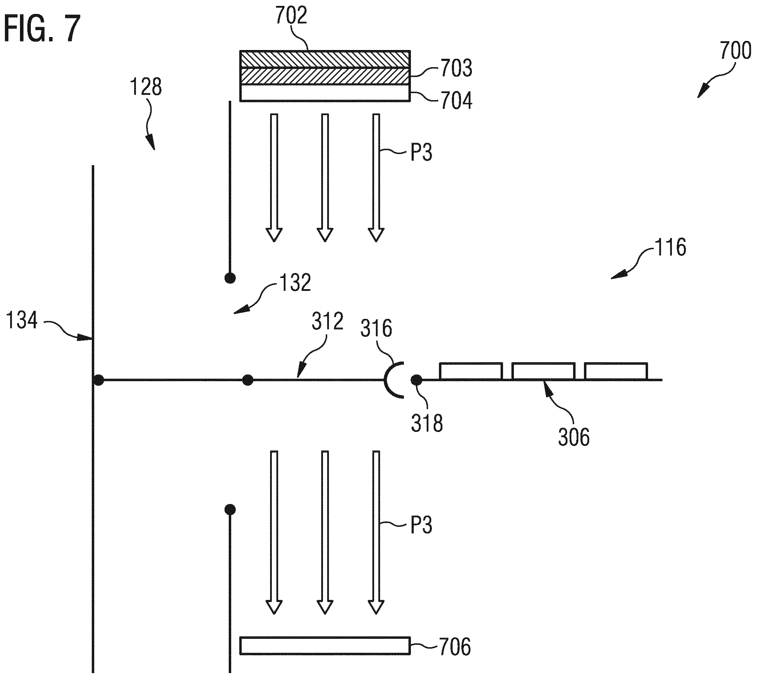

Figure 7 is a schematic side view of the sample carrier 306 handling unit 134 and the incubation chamber 116 of a microscope system 700 according to an embodiment.

In the present embodiment, the microscope system 700 comprises a fan assembly 702 configured to blow atmosphere into the incubation chamber 116 through a first opening 704 arranged on a top side of the sample chamber 104. A filter 703 is arranged between the fan assembly 702 and the first opening 704 in order to clean the atmosphere blown into the incubation chamber 116 from any contaminants. The atmosphere blown into the incubation chamber 116 exits the incubation chamber 116 via a second opening 706 arranged at a bottom side of the incubation chamber 116. This arrangement creates a laminar flow inside the incubation chamber 116, in particular next to the second opening 132 connecting the incubation chamber 116 to the transfer chamber 128, and next to the door 120 of the incubation chamber 116 used for accessing the incubation chamber 116 from the outside. The laminar flow is illustrated in Figure 7 by a third set of arrows P3. The laminar flow acts as a shield or curtain that prevents an intrusion of particles and atmosphere into the incubation chamber 116, and simultaneously prevents atmosphere from inside the incubation chamber 116 to escape. When a user moves their arm into the incubation chamber 116 via the door the laminar flow is only interrupted in a small region around the user's arm such that the remaining flow of atmosphere still acts as a protective shield. Further, any contamination brought in by the user's arm can be entrained by the flow of atmosphere and transported to the second opening 706 at the bottom side of the incubation chamber 116. A similar setup can be used to create a laminar flow of atmosphere inside the sample chamber 104.

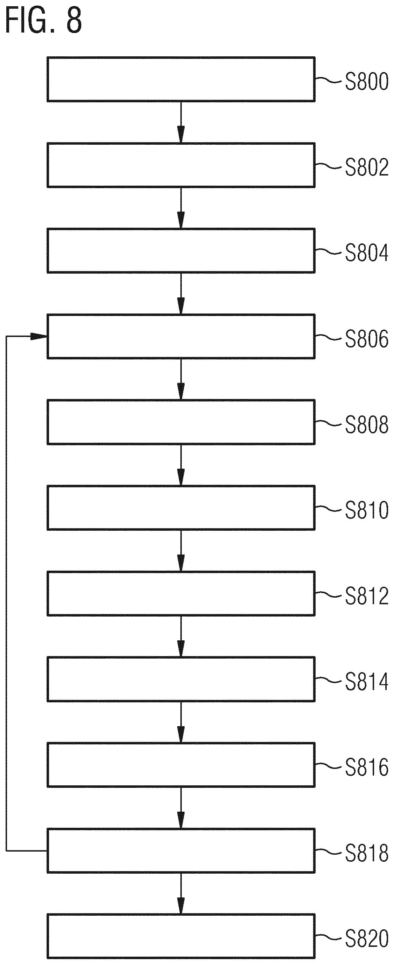

Figure 8 is a flowchart of a method for examining samples with the microscope system 100, 300, 600, 700 detailed above.

In step S800 the process is started. In an optional step S802 multiple sample vessels, for example petri dishes or microscope slides, containing the samples are loaded into the sample carrier 306. If the sample carrier 306 is a multiwell plate or similar, this step is not needed. In step S804 at least one sample carrier 306 containing the samples to be observed is loaded into the incubation chamber 116. The sample carrier 306 may be placed manually or automatically by means of an automated loader.

In step S806 a command to move the sample carrier 306 from its storing position 118 to the examining position 106 is given to the microscope system 100, 300, 600, 700 either by user input or a control unit, for example a computer, that performs an automated workflow. Whether or not a particular sample carrier 306 is to be moved, can in particular be determined based on an input from the sample condition determination unit 310.

In step S808 the laminar flow inside the incubation chamber 116 is initiated and the second door 400 between the transfer chamber 128 and the incubation chamber 116 is opened. Then the arm 312 moves to its lower position, extends into the incubation chamber 116 and grabs the sample carrier 306 that is to be moved. Once the arm 312 has secured the sample carrier 306, the arm 312 retracts into the transfer chamber 128. The second door 400 is closed and the laminar flow inside the incubation chamber 116 is stopped.

In step S810 the laminar flow inside the sample chamber 104 is initiated and the first door 322 between the sample chamber 104 and the incubation chamber 116 is opened. Then the arm 312 moves to its upper position, extends into the sample chamber 104 and positions the sample carrier 306 onto the microscope stage 324. When the sample carrier 306 is in the examining position 106, the arm 312 retracts into the transfer chamber 128. The first door 322 is closed and the laminar flow inside the sample chamber 104 is stopped.

In step S812 the microscopic examination of the sample or sample arranged in the sample carrier 306 takes place. When the examination has finished, a command to move the sample carrier 306 from the examining position 106 back to its storing position 118 is given to the microscope system 100, 300, 600, 700 in step S814 either by user input or the control unit.

In step S816 the laminar flow inside the sample chamber 104 is initiated and the first door 322 between the sample chamber 104 and the incubation chamber 116 is opened. Then the arm 312 moves to its upper position, extends into the sample chamber 104 and grabs the sample carrier 306 that is to be moved. Once the arm 312 has secured the sample carrier 306, the arm 312 retracts into the transfer chamber 128. The first door 322 is closed and the laminar flow inside the sample chamber 104 is stopped.

In step S818 the laminar flow inside the incubation chamber 116 is initiated and the second door 400 between the transfer chamber 128 and the incubation chamber 116 is opened. Then the arm 312 moves to its lower position, extends into the incubation chamber 116 and positions the sample carrier 306 in its storing position 118. When the sample carrier 306 is in its storing position 118, the arm 312 retracts into the transfer chamber 128. The second door 400 is closed and the laminar flow inside the incubation chamber 116 is stopped.

The steps S806 to S818 may be repeated for different sample carriers 306 stored inside the incubation chamber 116. In particular, the same sample carrier 306 may be examined multiple times in order to study the development of the sample or samples contained therein over a period of time. During the steps S806 to S818 the sample carrier 306 is continuously incubated.

In an optional step S820 at least one sample carriers 306 is removed from the sample chamber 104. If another experiment is performed at a later time, the sample carriers 306 may remain inside the incubation chamber 116. In step S822 the process is ended.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of Reference Signs

- 100: Microscope system
- 102: Housing
- 104: Sample chamber
- 106: Examining position
- 108: Door
- 110: Climate control unit
- 112: Optical table
- 114: Housing
- 116: Incubation chamber
- 118: Storing position
- 120: Door
- 122: Climate control unit
- 124: Optical table
- 126: Sample carrier transfer unit
- 128: Transfer chamber
- 130,132: Opening
- 134: Sample carrier handling unit
- 300: Microscope system
- 302: Housing
- 304: Shelf-like structure
- 306: Sample carrier
- 308: Camera
- 310: Sample condition determination unit
- 312: Arm
- 314: Guide rail
- 316: Engaging portion
- 318: Adapter portion
- 320: Sterilization means
- 322: Door
- 324: Microscope stage
- 326: Receiving portion
- 328: Imaging optics
- 330: Component space
- 332: Illumination system
- 400: Door
- 600: Microscope system
- 602: Guiding portion
- 700: Microscope system
- 702: Fan assembly
- 703: Filter
- 704,706: Opening
- P1, P2, P3: Arrows

## Claims

1. A microscope system (100, 300, 600, 700), comprising:
an enclosed sample chamber (104) configured to receive a sample carrier (306) in an examining position (106) in which a sample arranged on the sample carrier (306) can be microscopically examined;
an enclosed incubation chamber (116) that is separated from the sample chamber (104) and configured to receive the sample carrier (306) in at least one storing position (118); and
a sample carrier transfer unit (126) comprising an enclosed transfer chamber (128) that is connected to the sample chamber (104) by a first opening (130), and to the incubation chamber (116) by a second opening (132), and a sample carrier (306) handling device (134) arranged within the transfer chamber (128) and configured to move the sample carrier (306) between the storing position (118) and the examination position (106).

2. The microscope system (100, 300, 600, 700) according to claim 1, wherein the sample carrier (306) handling device (134) comprises an arm (312) that is movable within the transfer chamber (128), the arm (312) having an engaging portion (316) configured to engage and disengage with the sample carrier (306) in order to move the sample carrier (306).

3. The microscope system (100, 300, 600, 700) according to claim 1 or 2, wherein the engaging portion (316) is configured to engage and disengage with an adapter portion (318) of the sample carrier (306) in order to move the sample carrier (306).

4. The microscope system (100, 300, 600, 700) according to any one of the preceding claims, wherein the arm (312) is configured to extend and retract along at least one direction.

5. The microscope system (100, 300, 600, 700) according to any one of the preceding claims, wherein the transfer chamber (128) comprises sterilization means (320) configured to disinfect and/or sterilize the microscope system (100, 300, 600, 700), in particular the sample carrier (306) handling device (134) and the inside of the transfer chamber (128).

6. The microscope system (100, 300, 600, 700) according to any one of the preceding claims, wherein the first opening (130) and/or the second opening (132) comprise an automatic door, in particular a sliding door.

7. The microscope system (100, 300, 600, 700) according to any one of the preceding claims comprising a first fan assembly (702) configured to blow atmosphere into the sample chamber (104) through at least one first opening (130) of the sample chamber (104), and at least one second opening (132) of the sample chamber (104); wherein the at least one first opening (130) and the at least one second opening (132) are arranged on opposite sides of the sample chamber (104), in particular a top side and a bottom side of the sample chamber (104).

8. The microscope system (100, 300, 600, 700) according to claim 7, wherein the first fan assembly (702), the at least one first opening (130) and the at least one second opening (132) of the sample chamber (104) are configured to generate a laminar flow inside the sample chamber (104).

9. The microscope system (100, 300, 600, 700) according to any one of the preceding claims comprising a second fan assembly (702) configured to blow atmosphere into the incubation chamber (116) through at least one first opening (130) of the incubation chamber (116), and at least one second opening (132) of the incubation chamber (116); wherein the at least one first opening (130) and the at least one second opening (132) are arranged on opposite sides of the incubation chamber (116), in particular a top side and a bottom side of the incubation chamber (116).

10. The microscope system (100, 300, 600, 700) according to claim 9, wherein the first fan assembly (702), the at least one first opening (130) and the at least one second opening (132) are configured to generate a laminar flow inside the incubation chamber (116).

11. The microscope system (100, 300, 600, 700) according to any one of the preceding claims comprising at least one sample condition determination unit (310), the sample condition determination unit (310) having an image acquisition unit arranged inside the incubation chamber (116) and being configured to capture at least one image of the sample carrier (306), and a control unit configured to determine the condition of one or more samples placed in the sample carrier (306) based on the image.

12. The microscope system (100, 300, 600, 700) according to any one of the preceding claims comprising a microscope stage (324) arranged below the sample chamber (104) having a receiving portion (326) configured to receive the sample carrier (306) and that defines the examining position (106).

13. The microscope system (100, 300, 600, 700) according to any one of the preceding claims, wherein the sample chamber (104), the incubation chamber (116) and/or the transfer chamber (128) contain an incubation atmosphere, a sterile atmosphere and/or a semi sterile atmosphere.

14. The microscope system (100, 300, 600, 700) according to any one of the preceding claims comprising a box-type microscope housing (302) defining the sample chamber (104), the housing having a door for providing access to the sample chamber (104).

15. The microscope system (300, 600, 700) according to claim 14, wherein the microscope housing (302) further defines the incubation chamber (116) and the transfer chamber (128); and wherein the housing has a second door (400) for providing access to the incubation chamber (116).
